(19) **Europäisches Patentamt / European Patent Office / Office européen des brevets**

(11) **EP 4 779 027 A1**

(12) **EUROPEAN PATENT APPLICATION**
published in accordance with Art. 153(4) EPC

(43) Date of publication:
**22.07.2026 Bulletin 2026/30**

(21) Application number: **24865545.8**

(22) Date of filing: **13.09.2024**

(51) International Patent Classification (IPC):
*C12P 21/02* (2006.01)    *C07K 5/00* (2006.01)
*C07K 7/06* (2006.01)    *C07K 7/08* (2006.01)
*C12P 19/00* (2006.01)

(52) Cooperative Patent Classification (CPC):
**C07K 5/00; C07K 7/06; C07K 7/08; C12P 19/00;
C12P 21/02**

(86) International application number:
**PCT/JP2024/032798**

(87) International publication number:
**WO 2025/058046 (20.03.2025 Gazette 2025/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.09.2023 JP 2023150170**

(71) Applicant: **Megmilk Snow Brand Co. Ltd.
Sapporo-shi, Hokkaido 065-0043 (JP)**

(72) Inventors:
• **FUKUDOME Hirofumi
Sapporo-shi, Hokkaido 065-0043 (JP)**
• **HIGUCHI Junichi
Sapporo-shi, Hokkaido 065-0043 (JP)**

• **MITSUTSUKA Sho
Sapporo-shi, Hokkaido 065-0043 (JP)**
• **YAMAGUCHI Toshiyuki
Sapporo-shi, Hokkaido 065-0043 (JP)**
• **GODA Masayuki
Sapporo-shi, Hokkaido 065-0043 (JP)**
• **SAKAI Fumihiko
Sapporo-shi, Hokkaido 065-0043 (JP)**
• **KOSUGI Tatsuya
Sapporo-shi, Hokkaido 065-0043 (JP)**
• **ISHIDA Yuko
Sapporo-shi, Hokkaido 065-0043 (JP)**
• **NONOYAMA Noriko
Sapporo-shi, Hokkaido 065-0043 (JP)**

(74) Representative: **Mathys & Squire
32 London Bridge Street
The Shard
London SE1 9SG (GB)**

(54) **METHOD FOR PRODUCING GLYCOPEPTIDE-CONTAINING COMPOSITION**

(57) The objective of the present invention is to provide a production method for preparing a glycopeptide-containing composition that requires a short production time and a small amount of enzyme and that is capable of fractionating glycopeptides having a certain sugar content in high yield and with good reproducibility. The production method provided is a method for producing a composition containing a glycopeptide, which comprises simultaneously carrying out the following two steps in one reactor: (1) contacting a composition containing a glycoprotein with an endoprotease and/or an exoprotease in a solution to obtain a reaction mixture solution; and (2) ultrafiltering the reaction mixture solution of (1) above.

[Fig. 1]

**Description**

TECHNICAL FIELD

The present invention relates to a method for producing a glycopeptide-containing composition. The invention relates particularly to a method for producing a glycopeptide-containing composition by fractionating a glycopeptide-containing composition from a raw material containing a glycoprotein using an enzyme and an ultrafiltration membrane.

BACKGROUND ART

In recent years, glycosylated peptides (hereinafter sometimes referred to as glycopeptides) have been gaining significant attention as a new food ingredient with health benefits. Glycopeptides are generally produced by treating glycosylated proteins (hereinafter sometimes referred to as glycoproteins) with endoproteases and/or exoproteases. Meanwhile, simply treating glycoproteins with endoproteases and/or exoproteases results in treated products containing large amounts of unglycosylated peptides along with glycopeptides. Hence, this production method can only produce compositions with very low contents of target glycopeptides. A new step of separating glycopeptides from peptides is therefore required.

A method for separating glycopeptides and peptides from each other using an ultrafiltration membrane has hitherto been reported.

Patent Document 1 discloses a production method for preparing a mixture of glycopeptides with a high sialic acid content (sialylglycopeptides) by: allowing a protease to act on purified κ-casein; terminating the enzymatic reaction by heat treatment; and fractionating the treated solution using an ultrafiltration membrane.

Patent Document 2 discloses the following method for preparing a sialylglycopeptide concentrate. First, a protease is allowed to act on a composition containing glycomacropeptide (GMP) produced during the cheese manufacturing process. The enzymatic reaction is then stopped by heat treatment, and the treated solution is fractionated using an ultrafiltration membrane with a molecular weight cutoff of 500 to 3,000. The method for producing a composition containing these glycopeptides (hereinafter sometimes referred to as a glycopeptide-containing composition) involves the following two steps carried out separately:

(1) a step of enzymatically digesting a protein and terminating the enzymatic reaction to obtain an enzymatically digested solution (in which the enzyme has already been inactivated); and
(2) a step of subjecting the enzymatically digested solution to an ultrafiltration membrane to fractionate glycopeptides.

In this method, each step requires approx. 1 to 10 hours, resulting in a long production time due to the combined time required for both steps. Also, to fractionate glycopeptides, glycoproteins must be sufficiently digested, requiring large amounts of expensive enzymes (see "Comparative Example 1" in Examples that will be described later). Furthermore, because glycopeptides and peptides have very similar molecular weights after the enzymatic reaction, fractionation by ultrafiltration is inefficient, and even if glycopeptides are concentrated to a certain extent, the yield remains at around 50% (see Comparative Example 2 in the Examples below).

Thus, conventional methods for fractionating glycopeptides have the drawbacks of: requiring a long production time; requiring a large amount of digesting enzyme; and producing low yields of the target glycopeptides. Meanwhile, to reproducibly fractionate glycopeptides with a certain sugar content, it is necessary to prevent the enzymatic reaction from proceeding during membrane treatment, which requires the process to be carried out in two steps (1) and (2) as described above.

PRIOR ART DOCUMENT

PATENT LITERATURE

Patent Document 1: JP 2980362 B2
Patent Document 2: WO 2019/044960 A

SUMMARY OF INVENTION

TECHNICAL PROBLEM

The objective of the present invention is to provide a method for producing a glycopeptide-containing composition that requires a short production time and a small amount of enzyme and that is capable of fractionating glycopeptides having a certain sugar content in high yield and with good reproducibility.

MEANS FOR SOLVING PROBLEM

The present inventors have conducted extensive research to solve the above-mentioned problems and have established a production method that simultaneously performs two steps that were conventionally separated. Specifically, the inventors have found that the problem can be solved by a method in which an enzymatic reaction step and a step using an ultrafiltration membrane are simultaneously performed in the same reactor. Furthermore, the inventors have found that glycopeptides can be recovered with good reproducibility, irrespective of the treatment volume or speed, by performing a concentration treatment using an ultrafiltration membrane, followed by diafiltration. This led to the completion of the present invention. Specifically, the present invention has the following configurations.

<1> A method for producing a glycopeptide-containing composition, comprising carrying out the following two steps simultaneously in a single reactor:

(1) carrying out an enzymatic reaction in a mixture solution obtained by contacting in a solution a composition containing a glycoprotein with an endoprotease and/or an exoprotease; and
(2) ultrafiltering the mixture solution of (1) above.

<2> The production method of <1>, wherein the step (2) comprises concentrating and/or diafiltering the mixture solution.
<3> The production method of <2>, wherein the step (2) comprises diafiltering after concentrating the mixture solution.
<4> The production method of <1>, wherein an ultrafiltration membrane used in the step (2) has a molecular weight cutoff of 150 or more and 3,000 or less, the ultrafiltration membrane being configured to separate glycopeptides into the concentrate and peptides into the permeate.
<5> The production method of <1>, wherein the glycopeptide comprises a glycan bound to a hydroxyl group of a serine and/or threonine residue of a peptide.
<6> The production method of <1>, wherein the glycopeptide comprises a glycan consisting of sialic acid and galacto-N-biose, the total amount of which is 4.9 wt% or more.
<7> The production method of <1>, wherein the glycopeptide has a molecular weight of 700 or more and 4,000 or less.
<8> The production method of <1>, wherein the amino acids constituting a peptide contained in the glycopeptide are at least one and at most sixteen.
<9> The production method of <1>, wherein the glycopeptide comprises one or more glycan structures selected from the group consisting of the following (a) to (e):

(a) Neu5Ac$\alpha$2-3Gal$\beta$1-3(Neu5Ac$\alpha$2-6)GalNAc$\alpha$1;
(b) Gal$\beta$1-3(Neu5Ac$\alpha$2-6)GalNAc$\alpha$1;
(c) Neu5Ac$\alpha$2-3Gal$\beta$1-3GalNAc$\alpha$1;
(d) Neu5Ac$\alpha$2-3Gal$\beta$1-3(O-Ac-Neu5Ac$\alpha$2-6)GalNAc$\alpha$1; and
(e) Neu5Ac$\alpha$2-3Gal$\beta$1-3(O-diAc-Neu5Ac$\alpha$2-6)GalNAc$\alpha$1.

<10> The production method of <9>, wherein among the glycan structures contained in the glycopeptide, (a) is 3.2 wt% or more, (b) is 0.6 wt% or more, and (c) is 0.7 wt% or more.
<11> The production method of <9>, wherein among the glycan structures contained in the glycopeptide, the proportion of (a) relative to the total weight of (a), (b), and (c) is 70% or more.
<12> A method for producing a glycopeptide-containing composition, comprising:
ultrafiltering a mixture solution of a composition containing a glycoprotein and an endoprotease and/or an exoprotease during an enzymatic reaction of the endoprotease and/or the exoprotease.
<13> The production method of <12>, wherein the ultrafiltering comprises concentrating and/or diafiltering the mixture solution.
<14> A glycopeptide-containing composition, wherein the glycopeptide comprises glycan structures of the following (a) to (c):

(a) Neu5Ac$\alpha$2-3Gal$\beta$1-3(Neu5Ac$\alpha$2-6)GalNAc$\alpha$1;

(b) Galβ1-3(Neu5Acα2-6)GalNAcα1; and

(c) Neu5Acα2-3Galβ1-3GalNAcα1,

wherein the composition contains 3.2 wt% or more of (a), 0.6 wt% or more of (b), and 0.7 wt% or more of (c).

<15> A glycopeptide-containing composition, wherein the glycopeptide comprises glycan structures of the following (a) to (c):

(a) Neu5Acα2-3Gal β1-3(Neu5Acα2-6)GalNAcα1;

(b) Galβ1-3(Neu5Acα2-6)GalNAcα1;and

(c) Neu5Acα2-3Galβ1-3GalNAcα1,

wherein the weight ratio satisfies the following relationship:

$$(a)/[(a)+(b)+(c)] \geqq 70\%.$$

<16> A glycopeptide-containing composition produced by the production method of any one of <1> to <13>.

ADVANTAGEOUS EFFECTS OF INVENTION

According to the present invention, by simultaneously carrying out a step of performing an enzymatic reaction in a mixture solution in which a composition containing a glycoprotein is contacted with an endoprotease and/or an exoprotease in a solution, and a step of subjecting the mixture solution obtained in this step to ultrafiltration in the same reactor, it has become possible to fractionate a glycopeptide-containing composition having a certain sugar content in high yield and with good reproducibility in a short time and with a small amount of enzyme. This has made it possible to stably provide glycopeptide-containing compositions with high sialic acid content at low cost, and also to provide a composition containing glycopeptides with specific glycan structures at a predetermined ratio.

BRIEF DESCRIPTION OF DRAWINGS

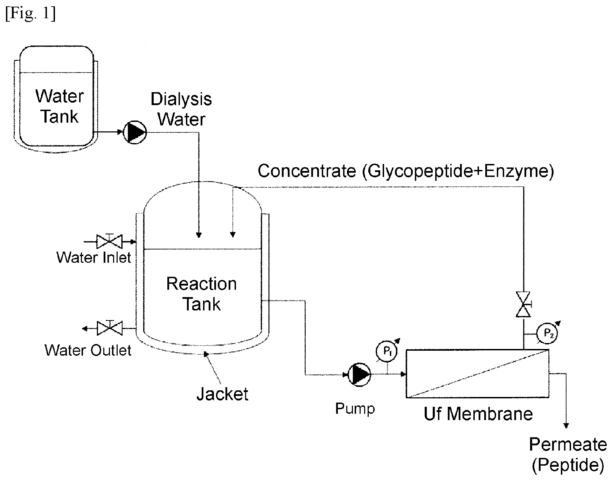

[Fig. 1] Fig. 1 is a schematic diagram of a bioreactor (reactor) used in a production method of the present invention.

DESCRIPTION OF EMBODIMENTS

(Method for Producing Glycopeptides)

A method for producing a glycopeptide-containing composition of the present invention is characterized by simultaneously carrying out the following two steps in one reactor:

(1) carrying out an enzymatic reaction in a mixture solution obtained by contacting in a solution a composition containing a glycoprotein with an endoprotease and/or an exoprotease; and
(2) ultrafiltering the mixture solution of (1) above.

The present invention is a method of ultrafiltering a solution in which an enzymatic reaction is in progress, rather than the conventional method of ultrafiltering a solution containing an inactivated enzyme after the enzymatic reaction is terminated by heating or the like to inactivate the enzyme. That is, the present invention differs from the conventional method in that a mixed solution of an active enzyme and a glycoprotein is ultrafiltered. In other words, the method for producing a glycopeptide-containing composition of the present invention is a method of ultrafiltering a solution in the midst of an enzymatic reaction between a composition containing a glycoprotein and an endoprotease and/or an exoprotease. Also, the method for producing a glycopeptide-containing composition of the present invention is a method for producing a composition containing glycopeptides with a specific composition of glycans by ultrafiltration of a mixed solution of a glycoprotein and a protease.

Fig. 1 shows a schematic diagram of a membrane bioreactor as an example of an apparatus for performing this process. A reaction tank is connected to an ultrafiltration membrane (sometimes called a UF membrane), and the concentrate side is piped so that it is returned to the reaction tank (also called a reactor). The reaction tank is also piped so that dialysis water is supplied from a water tank, thereby controlling the number of diafiltration runs. The permeate side of the UF membrane is configured to be discharged. The reaction tank is covered with a jacket, and hot water heated by steam is introduced into the jacket through a water inlet and circulated within the jacket. The circulated hot water is also discharged through a water outlet. This allows the temperature inside the tank to be controlled. Note that the temperature can also be controlled by other temperature control mechanisms, such as a heat exchanger.

When a composition containing glycoprotein, protease, and water as raw materials are supplied to the reaction tank and mixed therein, a protease reaction cleaves glycopeptides from the glycoprotein. Since the reaction tank is configured so that the concentrate is returned to the reaction tank via a UF membrane, the glycoprotein in the returned concentrate reacts with the protease again in the tank, resulting in further cleavage of glycopeptides. Because the UF membrane has a certain molecular weight cutoff, the supply of the concentrated protease reaction solution allows peptides with low molecular weights in the reaction solution to pass through the membrane, while glycopeptides with molecular weights above the certain level that are unable to pass through are returned to the tank. Repeated circulation allows glycopeptides of the desired molecular weight to be concentrated on the concentrate side (concentration process).

The concentration factor (CF) of the mixture solution is preferably greater than 1, and more preferably greater than or equal to 2. This is because a concentration factor of 2 or greater improves the reproducibility of the sialic acid content and sialic acid recovery rate irrespective of the treatment amount or treatment speed.

Furthermore, in the step (2) of the present invention, diafiltration (DF, also referred to as diafiltration) is preferably performed using a UF membrane. In step (2), both the concentration and diafiltration are preferably performed using the same UF membrane. In this case, diafiltration is preferably initiated after the concentration using the UF membrane. "Initiating after concentration" does not mean that dialysis is initiated after the concentration is fully completed, but also includes the case where dialysis is initiated after concentration has progressed to a certain extent. For example, if the final concentration is three times, dialysis may be initiated when the concentration has progressed twofold. The dialysis step is initiated by introducing dialysis water from the water tank into the reaction tank. Dialysis water and concentrate are supplied to the UF membrane from the reaction tank, and repeated circulation between the reaction tank and the UF membrane causes glycopeptides to migrate to the DF retentate (concentrate) side and peptides to migrate to the permeate side, allowing glycopeptides with a high sialic acid content to be fractionated into the DF retentate (concentrate) side.

The more times the DF is performed, the higher the sialic acid content on a solid basis and the smaller the variation thereof, but the lower the recovery rate of sialic acid. Therefore, it is desirable to perform the DF at a number of times that keeps these values (i.e., the sialic acid content, the variation, and the recovery rate) within the desired range.

According to conventional methods, an enzymatic reaction is carried out in a first reaction tank, the enzyme is inactivated by heating to terminate the enzymatic reaction, and the enzymatically reacted solution is supplied to a second tank for UF membrane treatment, which requires a total time for the enzymatic reaction and the UF membrane treatment. In contrast, according to the production method of the present invention, the enzymatic reaction is carried out in a single reaction tank, and the reaction solution undergoes UF membrane treatment as the reaction progresses. This allows the enzymatic reaction and UF membrane treatment to be carried out simultaneously in a single reactor. This saves time during the time-consuming enzymatic reaction treatment. Furthermore, by circulating the enzyme while mixing the enzyme with unreacted glycoprotein without inactivating the enzyme, it is possible to carry out the enzymatic reaction with a smaller amount of enzyme, which is an additional advantage.

The UF membrane used in step (2) above may be any membrane capable of separating the glycopeptide of the present invention into the concentrate, and preferably has a molecular weight cutoff of 150 or more and less than 5000. The molecular weight cutoff is more preferably 150 or more and 4000 or less, even more preferably 150 or more and 3000 or less, yet even more preferably 300 or more and 3000 or less, and most preferably 600 or more and 3000 or less.

(Protease)

The type of protease used in the production of the glycopeptide-containing composition of the present invention is not particularly limited as long as it can hydrolyze peptide bonds to obtain glycopeptides having the above molecular weights and glycans, and one or more types of endoproteases and exoproteases can be used. Enzymes usable in the production of food and pharmaceuticals are preferred, and examples include Actinase E (Kaken Pharma), Actinase AS (Kaken Pharma), Nucleisin (HBI), Orientase AY (HBI), Sumiteam FP (Shin-Nihon Chemical Industry), Sumiteam SPP-G (Shin-Nihon Chemical Industry), Protease A (Amano Enzyme), Peptidase R (Amano Enzyme), Alcalase (Novozyme), and Flavorzyme (Novozyme), which can be used alone or in combination.

The amount of protease added varies depending on the time required for degradation of glycoprotein, the type of glycoprotein, and other reaction conditions, operating conditions, etc. Specifically, the amount of protease added may be 0.1 to 20 wt% on a raw material basis, preferably 1 to 10 wt%, and more preferably 1 to 5 wt%. When whey protein isolate (WPI) is used as the raw material, the amount is preferably 1 to 10 wt%, more preferably 1 to 5 wt%, and even more preferably 1 to 3 wt% on a WPI-weight basis. The production method of the present invention has the advantage that a significantly smaller amount of enzyme is required to obtain a glycopeptide-containing composition having the same sialic acid content, compared to conventional methods in which the enzymatic reaction step and the dialysis treatment step using an ultrafiltration membrane are carried out separately.

(Composition Containing Glycoprotein)

The raw material used in the method for producing the glycopeptide-containing composition of the present invention may be any composition containing glycoprotein, including milk-derived raw materials such as whey materials such as cheese whey, whey protein concentrate, and whey protein isolate, casein materials, GMP materials, etc. The GMP can be any GMP obtained from whey derived from cow's milk, goat's milk, sheep's milk, or other animal milk.

(Glycopeptide-Containing Composition)

The glycopeptide-containing composition of the present invention is a composition containing glycopeptides fractionated by the above production method, and may also contain components other than glycopeptides (for example, peptides).
The above production method of the present invention enables efficient fractionation of glycopeptides having a sialic acid content of at least a certain amount, preferably 3.0 wt% or higher, more preferably 10.0 wt% or higher, and even more preferably 15.0 wt% or higher, in the glycopeptide-containing composition (on a solid basis).
More specifically, glycopeptides having one or more of the following features are included.

(i) The molecular weight is 700 or more and 4000 or less.
(ii) The glycan is bound to the hydroxyl group of the threonine and/or serine residue of the peptide.
(iii) The glycans contained in the glycopeptide are composed of sialic acid and galacto-N-biose, and the total amount of these is 4.9 wt% or more in the glycopeptide composition (on a solid basis).
(iv) The peptide chain consists of from 1 to 16 amino acid residues.

In a more specific aspect, the glycopeptide having such features is a peptide having a threonine and/or serine residue and a glycan bound to the hydroxyl group of the residue, and is a glycopeptide wherein the glycan is one or more selected from the group consisting of the following (a) to (e):

(a) Neu5Ac$\alpha$2-3Gal$\beta$1-3(Neu5Ac$\alpha$2-6)GalNAc$\alpha$1
(b) Gal$\beta$1-3(Neu5Ac$\alpha$2-6)GalNAc$\alpha$1
(c) Neu5Ac$\alpha$2-3Gal$\beta$1-3GalNAc$\alpha$1
(d) Neu5Ac$\alpha$2-3Gal$\beta$1-3(O-Ac-Neu5Ac$\alpha$2-6)GalNAc$\alpha$1
(e) Neu5Ac$\alpha$2-3Gal$\beta$1-3(O-diAc-Neu5Ac$\alpha$2-6)GalNAc$\alpha$1

Since the glycopeptides of the present invention are peptides containing one or more of the above glycans, they may also be peptides containing two or more of the above glycans, including peptides containing two or more of the same type of the above glycan, or peptides containing two or more different types of the above glycans. For example, the glycopeptides may contain two of (a), or may be glycopeptides containing (a) and one or more selected from the group consisting of (b) to (e).
Furthermore, the glycopeptide-containing composition of the present invention may be a mixture of peptides containing each of said glycans, and is preferably a glycopeptide mixture containing all of said glycans.
The production method of the present invention can provide a glycopeptide-containing composition containing the glycans (a) to (c) described above, wherein the composition contains 3.2 wt% or more of (a), 0.6 wt% or more of (b), and 0.7 wt% or more of (c).
The production method of the present invention can also provide a glycopeptide-containing composition containing the glycans (a) to (c), wherein the weight ratio of the glycopeptide-containing composition satisfies the following relationship:

$$(a)/[(a)+(b)+(c)] \geq 70\%$$

(Food, Beverages, and Pharmaceuticals That Contain Glycopeptide-Containing Composition)

The glycopeptide-containing composition obtained by the above production method of the present invention can be used in its intact form as a raw material or an ingredient for food and beverages, and the food and beverages can be manufactured according to the respective standard methods, except for adding the glycopeptide-containing composition.
Therefore, the glycopeptide-containing composition of the present invention may be incorporated into any food or beverages, or may be added to raw materials during the manufacturing process of the food or beverages. Examples of food and beverages include, but are not limited to, dairy products such as cheese, fermented milk, dairy lactic acid bacteria beverages, lactic acid bacteria beverages, butter, and margarine, beverages such as milk-based beverages, fruit beverages, and soft drinks, jelly, candy, processed egg products such as pudding, and mayonnaise, sweets and breads such as butter cake, various types of powdered milk, infant foods, and nutritional compositions.

The glycopeptide-containing composition obtained by the above production method of the present invention can be used in its intact form as a raw material for pharmaceuticals, and the pharmaceuticals can be manufactured by standard methods for tablets, capsules, powders, syrups, etc., except for adding the glycopeptide-containing composition.

Therefore, when formulating a pharmaceutical containing the glycopeptide-containing composition of the present invention as an active ingredient, the pharmaceutical can be formulated by appropriately mixing the composition with excipients, stabilizers, taste-masking agents, etc. that are approved for pharmaceutical use. In addition, the glycopeptide-containing composition can be dried as is and used as a powder or a coarse powder. It can also be formulated by mixing the composition with excipients, binders, disintegrants, lubricants, taste-and odor-masking agents, suspending agents, coating agents, and any other drugs. Possible dosage forms include tablets, capsules, granules, coarse powders, powders, and syrups.

EXAMPLES

A method for analyzing each sugar in the glycopeptide-containing composition of the present invention will be described below.

1. Method for Measuring Sialic Acid Content in a Glycopeptide-Containing Composition

The sialylglycopeptides and sialidase (Cosmo Bio Co., Ltd.) were dissolved in 50 $\mu$L of 50 mM potassium phosphate buffer (pH 5.0) so that the concentration of sialylglycopeptides is 250 $\mu$g/mL and the concentration of sialidase is 0.2 U/mL, respectively. The solution was incubated at 37°C for 24 hours and then diluted 2 to 10 times with ultrapure water, and the concentration of liberated sialic acid was analyzed by HPLC.

<HPLC Analysis Conditions>

· Equipment: DIONEX ICS-5000DP system (Thermo Fisher Scientific Co., Ltd.)
· Column: CarboPac PA1
· Detector: Electrochemical detector (pulsed amperometric mode)
· Mobile phase: ultrapure water (liquid A), 200 mM sodium hydroxide solution (liquid B), 100 mM sodium hydroxide solution containing 600 mM sodium acetate (liquid C)
· Flow rate: Liquids were passed at 1 mL/min.
· Concentration gradient:
For the first 7 minutes, the concentration of liquid B was 45% and liquid C was 10%, and in 10 minutes, liquid B was linearly decreased to 37.5% and liquid C was linearly increased to 25%. From 10 to 20 minutes, 37.5% liquid B and 25% liquid C were passed, and from 20.1 to 25 minutes, 45% liquid B and 10% liquid C were passed.

The amount of sialic acid in the sample was calculated from a calibration curve created using standard samples of known concentrations.

2. Measurement of Galacto-N-Biose (GNB) Content in the Glycopeptide-Containing Composition

Enzymes and a substrate were dissolved in 50 $\mu$L of 50 mM potassium phosphate buffer (pH 5.0) so that the concentration of sialylglycopeptides was 250 $\mu$g/mL, the concentration of sialidase (Cosmo Bio Co., Ltd.) was 0.2 U/mL, and the concentration of O-glycanase (ProZyme, Inc.) was 0.125 U/mL.

The lysate was incubated at 37°C for 24 hours using a thermal cycler (Takara Bio Inc.), and then the reaction was stopped by cooling on ice. The reaction solution was diluted 2 to 10 times with ultrapure water, and then the concentration of liberated GNB was analyzed by HPLC.

<HPLC Analysis Conditions>

· The instrument, column, detector, mobile phase, and mobile phase flow rate were the same as those for the measurement of sialic acid.

· Concentration gradient:

After elution with 50% solution B for the first 10 minutes, the proportion of solution C was increased linearly from 0% to 100% in 25 minutes, and elution was continued with 100% solution C until 30 minutes. Next, solution B was set to 50% at 30.1 minutes, and elution was continued with 50% solution B until 45 minutes.

GNB amount in the sample was calculated from a calibration curve created using standard samples of known concentrations.

[Comparative Example 1]

Preparation of Glycopeptides by Separately Carrying Out Enzymatic Reaction Treatment and Diafiltration Treatment Using an Ultrafiltration Membrane

1. Preparation of Glycopeptide-Containing Compositions

1-1. Enzymatic Reaction Treatment

3.0 kg of cheese whey-derived WPI (Provon (registered trademark) 190, Glanbia plc plc) was dissolved in 27.0 kg of filtered water to a concentration of 10% (w/w). The solution was heated to 55°C in a water bath. To the heated 10% Provon 190 solution, Alcalase (Novozymes) as an endoprotease and Flavorzyme (Novozymes) as an exoprotease were added. Both enzymes were added at 1% (w/w), 3% (w/w), or 5% (w/w) on a WPI-weight basis. After keeping at 55°C for 8 hours, the enzyme activity was inactivated by keeping at 85°C for further 30 minutes, and 30 kg of enzymatically digested solutions with three different enzyme addition levels were prepared.

1-2. Diafiltration Treatment Using Ultrafiltration Membranes

Using a membrane treatment system equipped with an organic UF membrane with a molecular weight cutoff of 1 kDa (hereinafter referred to as organic 1 kDa UF membrane) (Synder Filtration, Inc., membrane area: 5.39 m$^2$), the enzymatically digested solution prepared in section 1-1 above was circulated through the membrane while being heated at 55°C. Diafiltration (DF) was started using the filtered water heated to 55°C as dialysis water, and the DF retentate was freeze-dried to obtain a glycopeptide-containing composition. The DF factor (number of DF runs) is the value obtained by dividing the volume of permeate by the volume of DF retentate (enzymatically digested solution retained in the DF membrane).

2. Evaluation of Glycopeptide-Containing Compositions

The dry weight and sialic acid content of each glycopeptide-containing composition obtained by each level of test were measured. The preparation conditions, sialic acid content on a dry-weight basis, and sialic acid recovery rate of each glycopeptide-containing composition are shown in Table 1.

[Table 1]

| Level | Number of DF runs | Sialic acid content on a solid basis % (w/w) | Sialic acid recovery rate % |
|---|---|---|---|
| Enzyme 1% | 10 | 5.5 | 98.0 |
| Enzyme 3% | 10 | 11.9 | 90.5 |
| Enzyme 5% | 10 | 17.8 | 49.0 |

[Comparative Example 2]

Preparation of Glycopeptides by Separately Carrying Out Enzyme Reaction Treatment and Diafiltration Treatment Using an Ultrafiltration Membrane (Confirmation of Reproducibility)

1. Enzymatic Reaction Treatment and Diafiltration Treatment Using Ultrafiltration Membrane

Except that the amount of enzymes added were 5% (w/w) on a WPI-weight basis and that an 8-fold DF was applied, the glycopeptides were prepared in the same manner as in Comparative Example 1. Three tests were performed under the same preparation conditions.

2. Test Results

The dry weight and sialic acid content of the glycopeptide-containing composition were measured. Table 2 shows the preparation conditions of the glycopeptide-containing composition, the sialic acid content on a dry-weight basis, the sialic acid recovery rate, and the variability (CV).

[Table 2]

| Number of DF runs | Sialic acid content on a solid basis | | Sialic acid recovery rate | |
|---|---|---|---|---|
| | % (w/w) | CV | % | CV |
| 8 | 16.4 ± 0.7 | 4.3 | 53.2 ± 2.6 | 4.8 |

(n=3)

[Example 1]

Preparation of Glycopeptides by Simultaneously Carrying Out Enzymatic Reaction Treatment and Ultrafiltration Membrane Treatment (Concentration and Diafiltration)

1. Method for Preparing a Glycopeptide-Containing Composition

(i) Cheese whey-derived WPI (Provon190, Glanbia plc) was dissolved in filtered water to a concentration of 10% (w/w).
Since the minimum retention capacity of the membrane treatment system is 20 kg, and taking into consideration the reduction in liquid volume due to concentration, 10% (w/w) WPI solution at the following seven levels of solution amount was subjected to membrane treatment.

(Level #1) 20kg
(Level #2) 40kg
(Level #3) 60kg
(Level #4) 100kg
(Level #5) 200kg
(Level #6) 400kg
(Level #7) 800kg

(ii) Using a membrane bioreactor (Fig. 1) equipped with an organic 1 kDa UF membrane (Synder Filtration, Inc., membrane area 5.39 m$^2$), the solutions prepared above at each level were circulated through the UF membrane while being heated at 55°C.
(iii) When the temperature of the circulated solution reached 55°C, Alcalase (Novozymes) as an endoprotease and Flavorzyme (Novozymes) as an exoprotease were added. Each enzyme was added at 5% (w/w) on a WPI-weight basis, and the concentrate was concentrated to 20 kg.
(iv) Diafiltration (DF) was performed using filtered water heated to 55°C as the dialysis water for DF until the sialic acid content on a solid basis exceeded 16%.
(v) The obtained DF retentate was freeze-dried to obtain a glycopeptide-containing composition. To check for variability, the test was performed three times under the same preparation conditions.

2. Evaluation of Glycopeptide-Containing Compositions

The dry weight and sialic acid content of the glycopeptide-containing compositions obtained by each test level were measured. The preparation conditions, sialic acid content on a dry-weight basis, sialic acid recovery rate, and variability (CV) of each glycopeptide-containing composition are shown in Table 3. The number of DF runs is a value obtained by dividing the volume of the permeate by the volume of the UF membrane concentrate in 1.(iii) above.
These results confirmed that when the CF (concentration factor) was 2 or higher, the variation in sialic acid content and sialic acid recovery rate was reduced (i.e., reproducibility was confirmed), and it was found that a higher CF enabled more stable production of peptide compositions with a high sialic acid content.

[Table 3]

| Level | CF | Number of DF runs | Sialic acid content on a solid basis | | Sialic acid recovery rate | |
|---|---|---|---|---|---|---|
| | | | % (w/w) | CV | % | CV |
| #1 | - | 6 | 16.8 ± 0.8 | 4.8 | 78.7 ± 9.4 | 12.0 |
| #2 | 2 | 5 | 16.6 ± 0.4 | 2.2 | 79.7 ± 4.5 | 5.6 |
| #3 | 3 | 5 | 17.1 ± 0.7 | 4.1 | 78.4 ± 3.5 | 4.5 |
| #4 | 5 | 4 | 16.5 ± 0.6 | 3.5 | 79.9 ± 3.5 | 4.4 |
| #5 | 10 | 4 | 17.2 ± 0.7 | 4.2 | 77.8 ± 3.0 | 3.9 |
| #6 | 20 | 3 | 17.0 ± 0.6 | 3.7 | 78.7 ± 2.8 | 3.5 |
| #7 | 40 | 2 | 16.6 ± 0.5 | 2.9 | 79.7 ± 2.6 | 3.3 |

(n=3)

[Example 2]

Effect of the Amount of Enzyme Added on the Preparation of Glycopeptides by Simultaneous Execution of Enzymatic Reaction Treatment and Ultrafiltration Membrane Treatment (Concentration and Diafiltration)

1. Method for Preparing a Glycopeptide-Containing Composition

(i) 40.0 kg of cheese whey-derived WPI (Provon190, Glanbia plc) was dissolved in 360.0 kg of filtered water to a concentration of 10% (w/w).
(ii) Same as Example 1.
(iii) The same as in Example 1, except that each enzyme was added at a concentration of 1% (w/w), 3% (w/w), or 5% (w/w), respectively, on a WPI-weight basis, resulting in a 20-fold concentration.
(iv) and (v) Same as Example 1.

2. Evaluation of Glycopeptide-Containing Compositions

The dry weight and sialic acid content of the glycopeptide-containing compositions obtained by each test level were measured. The preparation conditions, sialic acid content on a dry-weight basis, sialic acid recovery rate, and variability (CV) of each glycopeptide-containing composition are shown in Table 4.

These results demonstrated that even with a low enzyme addition amount of 1% (w/w) relative to the raw material WPI, glycopeptide-containing compositions with a high sialic acid content of 15% (w/w) or more could be reproducibly fractionated. In the conventional method shown in Comparative Example 1, a glycopeptide-containing composition with a sialic acid content of only 5.5% (w/w) was obtained with a 1% (w/w) enzyme amount even after an 8-hour enzyme reaction. To obtain a sialic acid content as high as that of Example 1, a 5% (w/w) enzyme amount was required. Therefore, it was found that the method of the present invention, in which diafiltration treatment using an ultrafiltration membrane is simultaneously performed while maintaining the enzyme reaction, can reduce the amount of expensive enzyme required.

[Table 4]

| Level | CF | Number of DF runs | Sialic acid content on a solid basis | | Sialic acid recovery rate | |
|---|---|---|---|---|---|---|
| | | | % (w/w) | CV | % | CV |
| Enzyme 1% | 20 | 2 | 17.4 ± 0.8 | 4.4 | 82.0 ± 3.0 | 3.6 |
| Enzyme 3% | 20 | 2 | 16.5 ± 0.7 | 4.3 | 82.0 ± 3.1 | 3.8 |
| Enzyme 5% | 20 | 3 | 17.0 ± 0.6 | 3.7 | 78.7 ± 2.8 | 3.5 |

(n=3)

[Example 3]

Preparation of Glycopeptides by Simultaneously Performing Enzymatic Reaction Treatment and Ultrafiltration Membrane Treatment (Concentration and Diafiltration) in the Same Container: Variation in Intermediate Steps and Setting of Content Range

1. Method for Preparing a Glycopeptide-Containing Composition

(i) 4.0 kg of cheese whey-derived WPI (Provon 190, Glan-A) was dissolved in 36.0 kg of filtered water to a concentration of 10% (w/w).
(ii) Same as Example 1.
(iii) The same as in Example 1, except that each enzyme was added to a concentration of 1% (w/w) on a WPI-weight basis, resulting in a two-fold concentration.
(iv) and (v) Same as Example 1.

2. Test Results

(1) Each glycopeptide-containing composition was sampled at each of 0 to 5 DF runs in the above preparation method, and the dry weight and sialic acid content were measured. The preparation conditions, sialic acid content on a dry-weight basis, sialic acid recovery rate, and variability (CV) of each glycopeptide-containing composition are shown in Table 5.
(2) For glycopeptide-containing compositions A (one DF run) and B (five DF runs), the galacto-N-biose content, molecular weight, and number of amino acid residues were measured in addition to sialic acid. The sialic acid and galacto-N-biose contents, molecular weight, and number of amino acid residues on a dry-weight basis of glycopeptide-containing compositions A and B are shown in Table 6.

According to these results, the more DF runs there were, the higher the sialic acid content of the glycopeptide-containing composition obtained, and it was found that by concentrating it two-fold, the recovery rate of sialic acid was reproducible even for glycopeptide-containing compositions from intermediate processes.

[Table 5]

| CF | Number of DF runs | Sialic acid content on a solid basis | | Sialic acid recovery rate | |
|---|---|---|---|---|---|
| | | % (w/w) | CV | % | CV |
| - | - | 1.2 ± 0.0 | 2.5 | 100.0 | - |
| 2 | 0 | 2.1 ± 0.1 | 4.7 | 97.3 ± 2.5 | 2.6 |
| 2 | 1 | 3.2 ± 0.2 | 4.7 | 93.5 ± 3.5 | 3.8 |
| 2 | 2 | 8.1 ± 0.2 | 2.5 | 89.9 ± 3.8 | 4.2 |
| 2 | 3 | 12.0 ± 0.4 | 3.3 | 86.3 ± 3.0 | 3.5 |
| 2 | 4 | 14.9 ± 0.6 | 4.2 | 82.9 ± 3.2 | 3.9 |
| 2 | 5 | 16.6 ± 0.4 | 2.2 | 79.7 ± 4.5 | 5.6 |

(n=3)

[Table 6]

| Sample | CF | Number of DF runs | Sugar content on a solid basis %(w/w) | | Molecular weight (Da) | Number of amino acid residues (n) |
|---|---|---|---|---|---|---|
| | | | Sialic acid | Galacto-N-biose | | |
| WPI | - | - | 1.2 | 0.8 | - | - |
| Glycopeptide A | 2 | 1 | 3.0 | 1.9 | 700~4,000 | 1~16 |

(continued)

| Sample | CF | Number of DF runs | Sugar content on a solid basis %(w/w) | | Molecular weight (Da) | Number of amino acid residues (n) |
|---|---|---|---|---|---|---|
| | | | Sialic acid | Galacto-N-biose | | |
| Glycopeptide B | 2 | 5 | 16.6 | 10.3 | 700~4,000 | 1~16 |

[Example 4]

Preparation from Cheese Whey

1. Method for Preparing a Glycopeptide-Containing Composition

(i) Camembert cheese whey (0.6% protein) was kept at 50°C for 30 minutes to dissolve lactose crystals. Then, casein fine particles were removed using a milk clarifier (AMC-100, Iwai Machinery Co., Ltd.) at $6,500 \times g$, and thereafter the whey was defatted using a separator. The resulting 800 kg of defatted Camembert cheese whey solution was subjected to membrane treatment.
(ii) Same as Example 1.
(iii) Same as Example 1, except that each enzyme was added to a concentration of 1% (w/w) on a protein-weight basis, resulting in a two-fold concentration.
(iv) and (v) Same as Example 1.

2. Evaluation of Glycopeptide-Containing Compositions

The dry weight, sialic acid content, and galacto-N-biose content of the obtained glycopeptide-containing composition were measured. The preparation conditions and the sialic acid content and galacto-N-biose content on a dry-weight basis of the glycopeptide-containing composition are shown in Table 7.

[Table 7]

| CF | Number of DF runs | Sugar content on a solid basis %(w/w) | |
|---|---|---|---|
| | | Sialic acid | Galacto-N-biose |
| 20 | 7 | 16.3 | 10.1 |

[Example 5]

Preparation of Glycopeptides by Simultaneous Execution of Enzymatic Reaction Treatment and Ultrafiltration Membrane Treatment (Concentration and Diafiltration): Range of Membrane Molecular Weight Cutoff

1. Method for Preparing a Glycopeptide-Containing Composition

(i) 4.0 kg of cheese whey-derived WPI (Provon190, Glanbia plc) was dissolved in 36.0 kg of filtered water to a concentration of 10% (w/w).
(ii) The same as in Example 1, except that five types of organic UF membranes (Levels #1 to #5) with different molecular weight cutoffs shown in Table 7 were used.
(iii) The same as in Example 1, except that each enzyme was added to a concentration of 1 wt% on a WPI-weight basis, resulting in a two-fold concentration.
(iv) 20 kg of the concentrate was subjected to DF five times using filtered water heated to 55°C as the dialysis water for DF.
(v) Same as Example 1.

2. Evaluation of Glycopeptide-Containing Compositions

The glycopeptide-containing compositions obtained by each test level were measured for the dry weight, sialic acid content, and galacto-N-biose content. The preparation conditions, and the sialic acid content and galacto-N-biose content on a dry-weight basis of each glycopeptide-containing composition are shown in Table 8.

According to these results, neither sialic acid nor galacto-N-biose was detected when the molecular weight cutoff was 5000, but the sialic acid content and galacto-N-biose content on a solid basis increased with increasing molecular weight cutoff when the molecular weight cutoff was 150 or more. Therefore, it was found that the molecular weight cutoff of the ultrafiltration membrane used is preferably 150 or more and less than 5000, and more preferably 150 or more and 3000 or less.

[Table 8]

| Level | Molecular weight cutoff (Da) | Sugar content on a solid basis %(w/w) | |
|---|---|---|---|
| | | Sialic acid | Galacto-N-biose |
| #1 | 150-300 | 7.2 | 4.5 |
| #2 | 300-500 | 12.6 | 7.9 |
| #3 | 600-800 | 16.6 | 10.4 |
| #4 | 3000 | 18.1 | 11.3 |
| #5 | 5000 | ND | ND |

[Example 6]

Structural Analysis of Glycopeptide-Containing Compositions and Measurement of Glycan Content

(1) Structural Analysis

For structural analysis, an Orbitrap mass spectrometer Q-Exactive (Thermo Fisher Scientific) connected to a high-performance liquid chromatograph (HPLC) UltiMate 3000 (Thermo Fisher Scientific) was used (LC-ESI-IT MS). InertSustain AQ-C18 ($\varphi$2.1 mm$\times$150 mm, GL Sciences) was attached as a separation colum.

The mobile phases used were a 2% acetonitrile solution containing 0.1% formic acid (Solution A) and a 90% acetonitrile solution containing 0.1% formic acid (Solution B). The glycopeptide-containing composition was dissolved in ultrapure water to a concentration of 100 $\mu$g/mL, and 10 $\mu$L was introduced into the HPLC. The mobile phase was passed at 200 $\mu$L/min. After sample introduction, the proportion of Solution B was kept at 0% for 5 minutes, after which the proportion of Solution B was increased linearly from 0% to 10% over 25 minutes. The proportion of Solution B was then increased linearly from 10% to 100% over 20 minutes.

The mass spectrometer performed MS measurements (m/z 200-2000, positive mode) and MS/MS measurements. For MS/MS measurements, five most intense ions in descending order were automatically selected as precursor ions, and the m/z values of the resulting product ions were observed after destruction at collision energies of 15, 30, and 45. The acquired MS/MS spectra were analyzed using Proteome Discoverer 2.2 (Thermo Fisher Scientific) with the Byonic (Protein Metrics) node.

The protein database used was a database consisting only of GMP, and the glycan database used included 10 types of glycans consisting of one molecule of N-acetylhexosamine (HexNAc), 0 to 1 molecules of hexose (Hex), 0 to 2 molecules of N-acetylneuraminic acid (Neu5Ac), and 0 to 2 O-acetyl groups (O-Ac).

The glycans identified as (HexNAc), (HexNAc)(Hex), or (HexNAc)(Hex)(Neu5Ac)$_2$ by Byonic analysis were assigned to GalNAc, Gal$\beta$1-3GalNAc, or Neu5Ac$\alpha$2-3Gal$\beta$1-3(Neu5Ac$\alpha$2-6)GalNAc, respectively, according to the previously reported glycan structure of GMP. Furthermore, the MS/MS spectrum of the glycans identified as (HexNAc)(Hex)(Neu5Ac) were manually confirmed and those glycans were assigned to Neu5Ac$\alpha$2-3Gal$\beta$1-3GalNAc or Gal$\beta$1-3(Neu5Ac$\alpha$2-6)GalNAc. Similarly, the MS/MS spectrum of O-acetyl-modified Neu5Ac (O-Ac-Neu5Ac or O,O'-diAc-Neu5Ac) was manually confirmed and those glycans were attributed to Neu5Ac$\alpha$2-3Gal$\beta$1-3(O-Ac-Neu5Ac$\alpha$2-6) GalNAc or Neu5Ac$\alpha$2-3Gal$\beta$1-3(O-diAc-Neu5Ac$\alpha$2-6)GalNAc.

(2) Measurement of Glycan Content

The glycans contained in the sialylglycopeptide-containing composition were measured using the EZGlyco O-Glycan Prep Kit (Sumitomo Bakelite Co., Ltd.). 20 $\mu$L of a 10 mg/mL sialylglycopeptide solution was mixed with 15 $\mu$L of a glycan-releasing reagent (releasing reagent A:releasing reagent B=5:2) and incubated at 37°C for 75 minutes. The released glycans were then captured with glycan-capturing beads, which beads were then washed with acetonitrile. Next, 50 μL of methanol/acetic acid/ultrapure water (9/2/9) containing 3.9 mg of 2-aminobenzamide (2-AB) and 0.039 mg of reducing reagent was added to the beads, and the glycan solution was recovered by centrifugation at 3,000×g for 1 minute. This solution was then incubated at 50°C for 2.5 hours, after which excess 2-AB was removed by washing with acetonitrile using a cleanup column. Ultrapure water was added to the cleanup column to recover 2-AB-labeled glycans, which were then analyzed by LC/MS/FL. The structure of the glycan at each peak was determined by MS measurement, and the amount of each glycan in the sample was quantified from the area value of the fluorescence measurement.

<LC/MS Analysis Conditions>

· Instrument: UltiMate 3000 (Thermo Fisher Scientific Co., Ltd.)
· Column: Glycanpac AXH-1 (2.1 mm×150 mm, particle size 3 μm, Thermo Fisher Scientific Co., Ltd.)
· Detector: Q Exactive (Thermo Fisher Scientific Co., Ltd.) and RS Fluorescence Detector (Thermo Fisher Scientific Co., Ltd.)
· Mobile phase: 50 mM ammonium formate (pH 4.4)/acetonitrile (17/83)
· Flow rate: 0.4 mL/min
· Column temperature: 40°C
· MS settings: electrospray voltage, 3.5 kV; heat capillary temperature, 250°C; sheath gas, 45; auxiliary gas, 10; scan range, m/z 200-1500; resolution, 70,000.

2. Structural Analysis Results

Table 9 shows the glycan structures of glycopeptides detected by LC/MS analysis. As a result, all of the detected glycopeptides were peptides carrying glycans containing sialic acid. Many of the detected glycopeptides had a glycan structure of Neu5Acα1-3Galβ1-3(Neu5Acα1-6)GalNAc with two sialic acid molecules bound (Glycan types 3, 4, 5, 6, and 7), but Neu5Acα1-3Galβ1-3GalNAc (Glycan type 1) and Galβ1-3(Neu5Acα1-6)GalNAc (Glycan type 2), which have one sialic acid molecule, were also detected. In addition, the O-acetyl forms of sialic acid, N,O-diacetylneuraminic acid (O-Ac-Neu5Ac, Glycan type 4) and N,O,O-triacetylneuraminic acid (O-diAc-Neu5Ac, Glycan type 5), were also detected. Glycopeptides with two glycans bound to one peptide chain (Glycan types 6 and 7) were also detected.

The peptide chains detected by MS/MS spectral analysis were all peptide chains contained in bovine glycomacropeptide. All detected glycopeptides contained at least one serine or threonine residue, and the peptide chain length ranged from one residue to 16 residues. The molecular weights of the glycopeptides ranged from 745 to 3577.

3. Measurement Results of Glycan Content

The results of measuring the amounts of Glycan types 1, 2, and 3 contained in the glycopeptides A and B by LC/MS/FL are shown (Table 10). In both glycopeptides, glycan type 1 was 0.74 wt% or more, glycan type 2 was 0.65 wt% or more, and glycan type 3 was 3.25 wt% or more. Furthermore, the proportion of glycan type 3 in these glycans was 70.0% or more.

[Table 9]

| Glycan type on peptide | Glycan chain | Glycan MN |
|---|---|---|
| 1 | Neu5Acα2-3Galβ1-3GalNAcα1-S/T | 657 |
| 2 | Galβi-3(Neu5Acα2-6)GalNAcα1-S/T | 657 |
| 3 | Neu5Acα2-3Galβ1-3(Neu5Acα2-6)GalNAcα1-S/T | 948 |
| 4 | Neu5Acα2-3Galβ1-3(O-Ac-Neu5Acα2-6)GalNAcα1-S/T | 990 |
| 5 | Neu5Acα2-3Galβ1-3(O-diAc-Neu5Acα2-6)GalNAcα1-S/T | 1032 |
| 6 | Neu5Acα2-3Galβ1-3(Neu5Acα2-6)GalNAcα1-S/T Neu5Acα2-3Galβ1-3GalNAcα1-S/T | 948+657 |
| 7 | Neu5Acα2-3Galβ1-3(Neu5Acα2-6)GalNAcα1-S/T Neu5Acα2-3Galβ1-3(Neu5Acα2-6)GalNAcα1-S/T | 948+948 |

[Table 10]

| Sample | CF | Number of DF runs | Glycan content on a solid basis %(w/w) | | | Glycan composition % |
|---|---|---|---|---|---|---|
| | | | Glycan type 1 | Glycan type 2 | Glycan type 3 | type3/type1+2+3 |
| WPI | - | - | 0.37 | 0.38 | 1.14 | 60.3 |
| Glycopeptide A | 2 | 1 | 0.74 | 0.65 | 3.25 | 70.0 |
| Glycopeptide B | 2 | 5 | 2.24 | 1.37 | 21.84 | 85.8 |

**Claims**

1. A method for producing a glycopeptide-containing composition, comprising carrying out the following two steps simultaneously in a single reactor:

   (1) carrying out an enzymatic reaction in a mixture solution obtained by contacting in a solution a composition containing a glycoprotein with an endoprotease and/or an exoprotease; and
   (2) ultrafiltering the mixture solution of (1) above.

2. The production method of claim 1, wherein the step (2) comprises concentrating and/or diafiltering the mixture solution.

3. The production method of claim 2, wherein the step (2) comprises diafiltering after concentrating the mixture solution.

4. The production method of claim 1, wherein an ultrafiltration membrane used in the step (2) has a molecular weight cutoff of 150 or more and 3,000 or less, the ultrafiltration membrane being configured to separate glycopeptides into the concentrate and peptides into the permeate.

5. The production method of claim 1, wherein the glycopeptide comprises a glycan bound to a hydroxyl group of a serine and/or threonine residue of a peptide.

6. The production method of claim 1, wherein the glycopeptide comprises a glycan consisting of sialic acid and galacto-N-biose, the total amount of which is 4.9 wt% or more.

7. The production method of claim 1, wherein the glycopeptide has a molecular weight of 700 or more and 4,000 or less.

8. The production method of claim 1, wherein the amino acids constituting a peptide contained in the glycopeptide are at least one and at most sixteen.

9. The production method of claim 1, wherein the glycopeptide comprises one or more glycan structures selected from the group consisting of the following (a) to (e):

   (a) Neu5Acα2-3Galβ1-3(Neu5Acα2-6)GalNAcα1;
   (b) Galβ1-3(Neu5Acα2-6)GalNAcα1;
   (c) Neu5Acα2-3Galβ1-3GalNAcα1;
   (d) Neu5Acα2-3Galβ1-3(O-Ac-Neu5Acα2-6)GalNAcα1; and
   (e) Neu5Acα2-3Galβ1-3(O-diAc-Neu5Acα2-6)GalNAcα1.

10. The production method of claim 9, wherein among the glycan structures contained in the glycopeptide, (a) is 3.2 wt% or more, (b) is 0.6 wt% or more, and (c) is 0.7 wt% or more.

11. The production method of claim 9, wherein among the glycan structures contained in the glycopeptide, the proportion of (a) relative to the total weight of (a), (b), and (c) is 70% or more.

12. A method for producing a glycopeptide-containing composition, comprising:
ultrafiltering a mixture solution of a composition containing a glycoprotein and an endoprotease and/or an exoprotease during an enzymatic reaction.

13. The production method of claim 12, wherein the ultrafiltering comprises concentrating and/or diafiltering the mixture solution.

14. A glycopeptide-containing composition, wherein the glycopeptide comprises glycan structures of the following (a) to (c):

(a) Neu5Ac$\alpha$2-3Gal$\beta$1-3(Neu5Ac$\alpha$2-6)GalNAc$\alpha$1;
(b) Gal$\beta$1-3(Neu5Ac$\alpha$2-6)GalNAc$\alpha$1;and
(c) Neu5Ac$\alpha$2-3Gal$\beta$1-3GalNAc$\alpha$1,

wherein the composition contains 3.2 wt% or more of (a), 0.6 wt% or more of (b), and 0.7 wt% or more of (c).

15. A glycopeptide-containing composition, wherein the glycopeptide comprises glycan structures of the following (a) to (c):

(a) Neu5Ac$\alpha$2-3Gal $\beta$1-3(Neu5Ac$\alpha$2-6)GalNAc$\alpha$1;
(b) Gal$\beta$1-3(Neu5Ac$\alpha$2-6)GalNAc$\alpha$1;and
(c) Neu5Ac$\alpha$2-3Gal$\beta$1-3GalNAc$\alpha$1,

wherein the weight ratio satisfies the following relationship:

$$(a)/[(a)+(b)+(c)] \geqq 70\%.$$

16. A glycopeptide-containing composition produced by the production method of any one of claims 1 to 13.

[Fig. 1]

## INTERNATIONAL SEARCH REPORT

| International application No. |
| --- |
| **PCT/JP2024/032798** |

**A. CLASSIFICATION OF SUBJECT MATTER**

*C12P 21/02*(2006.01)i; *C07K 5/00*(2006.01)i; *C07K 7/06*(2006.01)i; *C07K 7/08*(2006.01)i; *C12P 19/00*(2006.01)i
FI: C12P21/02 B; C12P19/00; C07K5/00; C07K7/06; C07K7/08

According to International Patent Classification (IPC) or to both national classification and IPC

**B. FIELDS SEARCHED**

Minimum documentation searched (classification system followed by classification symbols)

C12P21/02; C07K5/00; C07K7/06; C07K7/08; C12P19/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2020-124126 A (SNOW BRAND MILK PRODUCTS CO., LTD.) 20 August 2020 (2020-08-20) | 14-16 |
| | claims, particularly claim 8, paragraphs [0017], [0018], paragraphs [0029], [0030] in example 1, table 2 | |
| Y | | 1-16 |
| X | WO 2019/044960 A1 (SNOW BRAND MILK PRODUCTS CO., LTD.) 07 March 2019 (2019-03-07) | 14-16 |
| | claims 16, 19, 22, paragraph [0020], paragraph [0025] in example 1, [0027] in example 3, [0029] in example 4, table 2 | |
| Y | | 1-16 |

☑ Further documents are listed in the continuation of Box C.   ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **25 November 2024** | **03 December 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
| --- |
| **PCT/JP2024/032798** |

**C. DOCUMENTS CONSIDERED TO BE RELEVANT**

| Category* | Citation of document, with indication, where appropriate, of the relevant passages | Relevant to claim No. |
| --- | --- | --- |
| Y | JP 56-123922 A (INSTITUT RECHERCHE AGRONOMIQUE) 29 September 1981 (1981-09-29)<br>claims 4, 5, page 3, upper left column, first - second paragraphs, page 8, lower left column, second paragraph - upper right column, first paragraph, page 10, lower right column, third paragraph, page 11, upper left column, second paragraph - upper right column, first paragraph, fig. 1, 2 | 1-16 |
| A | US 2013/0267683 A1 (NESTEC S. A.) 10 October 2013 (2013-10-10)<br>claims, fig. 2 | 1-16 |

Form PCT/ISA/210 (second sheet) (July 2022)

**INTERNATIONAL SEARCH REPORT**
Information on patent family members

International application No.

**PCT/JP2024/032798**

| Patent document cited in search report | | | Publication date (day/month/year) | Patent family member(s) | | | Publication date (day/month/year) |
|---|---|---|---|---|---|---|---|
| JP | 2020-124126 | A | 20 August 2020 | (Family: none) | | | |
| WO | 2019/044960 | A1 | 07 March 2019 | EP | 3677127 | A1 | |
| | | | | claims 16, 19, 22, paragraphs [0038], [0039], paragraphs [0051], [0052] in example 1, [0059] in example 3, [0066] in example 4, table 2 | | | |
| JP | 56-123922 | A | 29 September 1981 | US | 4358465 | A | |
| | | | | claims 4, 5, column 1, lines 5-23, column 8, lines 24-41, column 11, line 27 - column 12, line 5, fig. 1, 2 | | | |
| | | | | EP | 34083 | A1 | |
| US | 2013/0267683 | A1 | 10 October 2013 | WO | 2012/085114 | A1 | |
| | | | | EP | 2468109 | A1 | |
| | | | | CN | 103269603 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2980362 B
- WO 2019044960 A